# EUROPEAN PATENT SPECIFICATION

(11) **EP 3 897 619 B1**
(45) Date of publication and mention of the grant of the patent: **23.11.2022**
(21) Application number: 19828734.4
(22) Date of filing: 19.12.2019
(51) Int. Cl.: A61K 31/366, A61K 45/06, A61P 21/06, A61P 21/00

(54) **USE OF CARNOSOL FOR INCREASING MUSCLE PROTEIN SYNTHESIS**
VERWENDUNG VON CARNOSOL ZUR STEIGERUNG DER MUSKELPROTEINSYNTHESE
UTILISATION DE CARNOSOL POUR AUGMENTER LA SYNTHÈSE DE PROTÉINES MUSCULAIRES

(30) Priority: 21.12.2018 EP 18306811
(43) Date of publication of application: 27.10.2021
(73) Proprietor: Centre national de la recherche scientifique, 75016 Paris (FR); UNIVERSITE DE MONTPELLIER, 34090 Montpellier (FR); INSTITUT NATIONAL DE LA SANTE ET DE LA RECHERCHE MEDICALE - INSERM, 75013 Paris (FR); Université Paul-Valery Montpellier 3, 34090 Montpellier (FR); Ecole Pratique des Hautes Etudes (EPHE), 75014 Paris (FR); Institut de Recherche pour le Développement, 13572 Marseille Cedex 02 (FR)
(72) Inventor: CARNAC, Gilles, 34000 MONTPELLIER (FR); SAINT, Nathalie, 34190 BRISSAC (FR); MOREL, Sylvie, 34080 MONTPELLIER (FR); RAPIOR, Sylvie, 34090 MONTPELLIER (FR); VITOU, Manon, 34470 PEROLS (FR)
(74) Representative: Gevers & Orès
(86) International application number: PCT/EP2019/086235
(87) International publication number: WO 2020/127681

(56) References cited:
- WO-A1-2006/101663
- WO-A1-2009/106125
- JP-B2- 5 884 112
- VLAVCHESKI FILIP ET AL: "Carnosol Increases Skeletal Muscle Cell Glucose Uptake via AMPK-Dependent GLUT4 Glucose Transporter Translocation.", INTERNATIONAL JOURNAL OF MOLECULAR SCIENCES 29 APR 2018, vol. 19, no. 5, 29 April 2018 (2018-04-29) , XP002792545, ISSN: 1422-0067

## Description

### FIELD OF THE INVENTION:

The present invention relates to the increase of muscle protein synthesis, the reduction of the muscle protein degradation, the prevention and/or the treatment of pathological or non pathological loss of muscular mass in a subject.

### BACKGROUND OF THE INVENTION:

In humans, the skeletal muscles represent around 45% of the body mass. Due to its ability to generate the strength, the muscle is responsible of mobility, breathing and posture. The skeletal muscles play also a key role as a regulator of metabolism by using a large quantity of glucose and lipids, in particular during exercise. Many pathological (cancer, diabetes...) or non pathological (sedentary lifestyle, bed rest, immobilization, stay in space...) conditions lead to a loss of muscle mass thereby reducing functional capacities and life expectancy.

Many data show the beneficial effect of the exercise which enables to reduce the decline in performance of muscle function. However the compliance to exercise therapy remains low and its implementation is difficult.

In order to preserve the muscular mass, new nutritional strategies could be an interesting alternative to exercise therapy. Supplementation with vitamin D has been reported to increase muscular strength. (Morley JE, Pharmacologic Options for the Treatment of Sarcopenia, Calcif Tissue Int. 2016 Apr;98(4):319-33). There are also evidences showing that a diet rich in proteins (1-1.2 g/kg/day) may also enhance the muscular mass and in a lower extent the muscular function. Thus, at present time, most commercially available dietary supplements able to slow the muscular loss and to restore muscular function are supplemented with antioxidants, protein or some amino acids. However, the efficiency of these dietary supplements is low.

JP 5 884112 B2 discloses a muscle enhancing composition comprising rosemary extract for increasing muscle mass in humans and animals.

WO 2006/101663 discloses a composition comprising rosemary extract for use in the prevention of loss of muscle and maintenance of muscle mass.

The compounds able to fight against muscular loss remain limited. Thus, there is a need for new efficient dietary supplements for increasing muscular mass.

### DETAILED DESCRIPTION OF THE INVENTION

Now, the inventors have found that carnosol has a hypertrophic effect on muscle. They have shown that carnosol stimulates the synthesis of protein in muscle cells while it inhibits the degradation of proteins.

A subject of the present invention is therefore the use of carnosol or of a composition comprising carnosol for increasing muscle protein synthesis in a subject.

In particular, the carnosol or the composition comprising carnosol of the invention may be capable of increasing muscle protein synthesis by at least 50%, 60%, 70%, preferably 80%, more preferably 90% or most preferably 100%, as compared to a negative control.

The synthesis of muscle protein may be measured *in vitro* by the method disclosed in Schmidt et al., SUnSET, a nonradioactive method to monitor protein synthesis, Nat Methods. 2009 Apr;6(4):275-7. Muscle cells were treated with puromycin, 30 minutes before recovering the protein from the cells. Then, a western blot is carried out with anti-puromycin antibodies in order to show the translation rate of the mRNA in the living cells.

The present invention also relates to the use of carnosol or of a composition comprising carnosol for reducing the muscle protein degradation in a subject.

In particular, the carnosol or the composition comprising carnosol of the invention may be capable of reducing the muscle protein degradation by at least 50%, 60%, 70%, preferably 80%, more preferably 90% or most preferably 100%, as compared to a negative control.

The muscle protein degradation may be measured by assaying the level of expression of an ubiquitin ligases such as muscle RING-finger protein-1 (MuRF1).

Carnosol (CAS 5957-80-2) is an ortho-diphenolic dipterpene having the following structure:

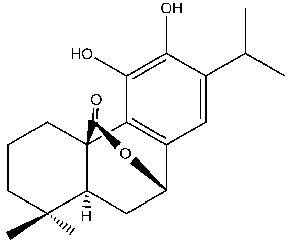

Carnosol has been first isolated in 1942 from the plant *Salvia carnosa* (purple sage). Subsequently, carnosol has been extracted from many other plant species including rosemary. Carnosol possesses a range of therapeutic effects such as anti-cancer, anti-inflammatory, and anti-oxidant activities (Kashyap D. et al., Mechanistic insight into carnosol-mediated pharmacological effects: Recent trends and advancements. Life Sci. 2017 Jan 15;169:27-36). However, until the discovery of the inventors, no hypertrophic effect of carnosol on muscle had been shown.

The carnosol for use according the present invention may be carnosol extracted from a plant extract, in particular extracted from rosemary, or synthetic carnosol.

### Composition comprising carnosol

The composition comprising carnosol may be a food supplement or a medicament

Typically, the composition comprising carnosol comprises an effective amount, preferably a therapeutically effective amount of active ingredient i. e. the carnosol together with an excipient.

An "effective amount" as used herein refers to that amount which is sufficient to provide a hypertrophic effect on muscle in the subject to whom it is administered. Muscle hypertrophic effect refers to an increase in the volume and/or the muscle mass. This increase in the volume and/or muscle mass may go along with a modification in muscle morphology or not.

The effect of the composition according to the invention can be readily verified by various assays, using appropriate animal models or muscle cells culture models (as disclosed for example in the below paragraph Examples). The effective dose is determined and adjusted depending on factors such as the composition used, the route of administration, the physical characteristics of the individual under consideration such as sex, age and weight, concurrent medication, and other factors, that those skilled in the medical arts will recognize. For example, it is well known within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Typically, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the subject to be treated. A medicament typically contains from about 0.01 mg to about 500 mg of the active ingredient, typically from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day. Moreover, in order to determine the effective dose, the person skilled in the art may notably rely on the studies on the toxicity of carnosol and its use as anticancer agent (Johnson JJ., Carnosol: a promising anti-cancer and anti-inflammatory agent. Cancer Lett. 2011 Jun 1;305(1):1-7 and Wang L., Carnosol suppresses patient-derived gastric tumor growth by targeting RSK2. Oncotarget. 2018 Feb 6;9 (76):34200-34212).

In one embodiment, the composition comprising carnosol comprises at least 0.1% w/w, preferably at least 0.5% w/w, more preferably at least 1%, at least 2%, at least 2.5%, at least 3%, at least 4%, at least 5% or at least 10% w/w of carnosol.

The compositions according to the invention are formulated for parenteral, transdermal, oral, rectal, intrapulmonary, subcutaneous, sublingual, topical or intranasal administration. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

In a preferred embodiment, the compositions according to the invention are formulated for oral or topical administration, more preferably oral administration.

The composition comprising carnosol may also comprise another active ingredient in addition to carnosol for example a compound selected from the group consisting of antioxidant, amino acid source and vitamin D.

As antioxidant any antioxidant may be used, preferably the antioxidant is selected from the group consisting of polyphenols, vitamins, carotenoids, trace elements and combinations thereof.

The vitamins may for example be vitamin E (tocopherol), vitamin A (retinol or beta-carotene) or vitamin C (ascorbic acid).

The trace element is preferably selenium.

Several spices or herbs such as oregano, cumin, ginger, garlic, coriander, onion, thyme, marjoram, tarragon, peppermint, cinnamon and/or basil may also be used as antioxidant as well as fruit extracts or dried fruits may be used. Examples of antioxidant fruit extracts or dried fruits are pears, apples, raisins, grapes, figs, cranberries, blueberries, blackberries, raspberries, strawberries, blackcurrants, cherries, plums, oranges, mango, and/or pomegranates.

Antioxidants may be used as purified compounds or partially purified compounds.

In an embodiment of the invention, the composition comprising carnosol also comprises vitamin D.

In another embodiment of the invention the composition comprising carnosol also comprises an amino acid source.

The amino acid source may be amino acids in free form or it may be peptides and/or proteins. The protein source may be dairy, animal or vegetable proteins.

In a preferred embodiment of the invention, the amino acid source is a protein selected from the group consisting of whey protein, casein protein, pea protein, soy protein, wheat protein, corn protein, or rice protein, proteins from legumes, cereals and grains in general or combinations thereof. The protein may also be selected from nuts and seeds. The amino acid source is preferably a whey protein.

In one alternative embodiment, the composition comprising carnosol does not comprise a compound selected from the group consisting of ursolic acid, vitamin D, an amino acid source, carnosic acid, rosmarinic acid, apple fruit derivative and eicosapentaenoic acid.

### Uses of carnosol or of a composition comprising carnosol

An object of the present inventionis to increase muscle proteins synthesis and/or to reduce the muscle protein degradation in a subject in need thereof comprising a step of administering to said subject an effective amount of carnosol or of a composition comprising carnosol.

Due to its properties on muscle protein synthesis and degradation, the carnosol may be used in many applications such as applications involving the prevention and/or the loss of muscle mass, in particular loss of skeletal muscle mass. Typically, carnosol may be used in therapeutic applications for example in order to treat and/or prevent muscle atrophy, in particular sarcopenia; in non therapeutic applications for example in a subject who has lost or have a risk of loss its muscle mass due to an immobilization or a stay in space but also in sportspersons in order to increase their performance. Finally, carnosol may be used in farming in order to increase the muscle mass of livestock animals.

The subject to whom is administered the carnosol or the composition comprising carnosol according to the various embodiments of the invention may be a human or a non human animal.

The subject may be an elderly or a young subject. In human, an elderly subject refers to a human over 60 years old and a young subject refers to a human under 30 years old. Preferably, the subject is an elderly subject.

In one embodiment, the subject is selected in the group consisting of a subject having a sedentary lifestyle, a subject at bed rest or having been at bedrest, an immobilized subject, an undernourished subject, a malnourished subject and an astronaut.

In another embodiment, the subject is a domestic animal, preferably livestock or poultry.

The use of carnosol according to the invention may be accompanied by physical exercise.

### Therapeutic applications of carnosol

An object of the invention is carnosol or a composition comprising carnosol for use in the prevention and/or the treatment of muscle atrophy in a subject.

The present invention also relates to carnosol or of a composition comprising carnosol for use for the prevention and/or the treatment of muscle atrophy and/or muscular dystrophy, preferably muscle atrophy. Muscular dystrophy may be selected from the group consisting of Duchenne muscular dystrophy, Becker muscular dystrophy, facioscapulohumeral muscular dystrophy, and myotonic dystrophy.

Muscle atrophy may be caused by many reasons. For example, it may result from several diseases, such as cancer, AIDS, congestive heart failure, COPD (chronic obstructive pulmonary disease), renal failure, trauma, sepsis, severe burns, mental disease such as anorexia, neuronal disease, cachexia, obesity and drug-related iatrogenia.

Muscle atrophy may also result in the disorder state sarcopenia, i.e. lost muscle mass, size, strength and functionality because of ageing. Thus in a preferred embodiment, carnosol or the composition comprising carnosol according to the invention is for use in the prevention and/or the treatment of sarcopenia in a subject.

The muscle atrophy may be of different grades, such as severe muscle atrophy as in extreme frailty elderly persons. These elderly persons will have difficulty in carry on every day activities and taking care of them self. Muscle atrophy, but of a less severe degree will allow some movement and some muscle activity, but insufficient to sustain the complete muscle tissue.

### Non therapeutic applications of carnosol

In addition to pathological causes, the loss of muscle mass may also result from disuse or insufficient use of the respective muscle. For example, it may result from lack of physical activity, such as from immobilization or hip-fracture recovery. Muscle atrophy may also be caused by insufficient or inappropriate nutrition or starvation. Thus, the present invention also relates to the use of carnosol or of a composition comprising carnosol for preventing and/or treating loss of muscle mass and/or for increasing muscle mass in a subject. Typically, the present invention relates to a method for preventing and/or treating loss of muscle mass and/or for increasing muscle mass in a subject in need thereof comprising a step of administering to said subject an effective amount of carnosol or of a composition comprising carnosol.

As mentioned above, the subject may be selected from the group consisting of a subject having a sedentary lifestyle, a subject at bed rest or having been at bedrest, an immobilized subject, an undernourished subject, a malnourished subject and an astronaut.

The subject may also be a sportsperson. Indeed, carnosol or composition comprising carnosol allows sportspersons to increase their performance.

### Use of carnosol in farming

Due to its effect on muscle protein synthesis, the carnosol may also have an application in farming.

Therefore, another object of the invention is the use of carnosol for increasing the muscle mass in livestock or poultry.

The present invention also relates to a method for increasing muscle mass of livestock animal comprising the step of administrating to said animal carnosol or a composition comprising carnosol. The composition comprising carnosol may be a feed comprising carnosol.

The invention will be further illustrated by the following figures and examples. However, these examples and figures should not be interpreted in any way as limiting the scope of the present invention.

### FIGURES

Figure 1 shows the hypertrophic activity of hydroalcoholic extract of Rosemary leaves on myotubes from young human subjects. Area of myotubes in a culture medium which comprises only culture medium (CTRL), or dry extract of a hydroalcoholic extract of Rosemary leaves at a concentration of 20 µg/mL (RL) are shown.
Figure 2 is a schematic diagram of the bioassay-guide fractionation of the extract of rosemary leaves (RL). The fractions having a hypertrophic activity are in grey.
Figure 3 shows the hypertrophic activity of commercially available carnosol on myotubes from young human subjects. Area of myotubes in a culture medium which comprises only culture medium (CTRL), 5 µg/ml of fraction C (fraction C), 5 µg/ml of fraction M (fraction M), 5 µg/ml of commercially available carnosol (CO) is shown.
Figure 4 shows the hypertrophic activity of carnosol (CO) on myotubes from young human subjects at various concentrations (0.25 µg/ml, 0.5 µg/ml, 1 µg/ml, 2.5 µg/ml, 5 µg/ml).
Figure 5 shows the hypertrophic activity of carnosol on myotubes of elderly subjects (subjects 1 and 2).
Figure 6 shows the effect of the administration of carnosol (CO) on gastrocnemius muscle of mice compared to a control (CTRL).
Figure 7 shows the effect of administration of carnosol (CO) on MURF1 protein level compared to a control (CTRL) in mice.
Figure 8 shows the hypertrophic activity on myotubes of a culture medium which comprises 2 µg/ml of carnosol, 2 µg/ml of carnosic acid, 2 µg/ml of rosmarinic acid, 2 µg/ml of ursolic acid and 50 µg/ml of vitamin C.
Figure 9 shows the hypertrophic activity on myotubes of carnosol (CO) and carnosic acid (CA). Area of myotubes in a culture medium which comprises only culture medium (CTRL), 1 µg/ml of carnosic acid (CA 1 µg/ml), 5 µg/ml of carnosic acid (CA 5 µg/ml), 1 µg/ml of carnosol (CO 1µg/ml), 5 µg/ml of carnosol (CO 5 µg/ml).
Figure 10 shows the hypertrophic activity on myotubes of 1 µg/ml of carnosol (CO 1µg) and 5mM of leucine (leucine 5mM).
Figure 11 shows the hypertrophic activity on myotubes of 1 µg/ml of carnosol (CO 1µg), 25µM of β-hydroxy-β-methylbutyrate (HMB 25 µM) and 100µM of β-hydroxy-β-methylbutyrate (HMB 100µM).
Figure 12 shows the hypertrophic activity on myotubes of 1 µg/ml of carnosol (CO 1µg), and 10⁻⁷M of vitamin D (Vit D 10⁻⁷M).
Figure 13 is a schematic diagram of the method used to determine if the protein synthesis is the pathway involved in the hypertrophic activity of carnosol. In figure 13 is also shown the western blot comparing the protein synthesis of myotubes exposed to carnosol compared to a control.
Figure 14 shows the level of puromycine, p-S6K/S6K, p-S6/S6, p-4EBP1/4EPB1, Murf1 after administration of carnosol.
Figure 15 shows the hypertrophic activity on myotubes of 1 µg/ml of carnosol (CO 1µg), 1 µg/ml of carnosic acid (CA 1 µg/ml), and 50µM of dimethylfumarate (DMF 50 µM).

### EXAMPLES

### Culture cell model

In order to assay the hypertrophic effect on muscle of compounds from vegetal extracts, the inventors have designed a culture cell model of human skeletal muscle satellite cells. The skeletal muscle satellite cells are able to proliferate and to differentiate *ex vivo.* When cultured in a growth factors rich medium, the skeletal muscle satellite cells proliferate in the form of myoblasts. At confluence, the myoblasts morphologically differentiate by fusing thereby producing long multinucleated cells (myotubes or myofibers) which express muscular protein. Myotubes are able to respond to hypertrophic and atrophic signals by modulating the balance between the protein synthesis (hypertrophy) or the protein degradation (atrophy) (EI Haddad M. et al., Cell Mol Life Sci. 2017 May;74(10):1923-1936).

The recovered skeletal muscle satellite cells used in the culture cell model by the inventors were recovered from a muscular biopsy of young and elderly subjects.

The satellite cells can be easily recovered from a muscle biopsy and cultured *ex vivo* in dishes where they proliferate as myoblasts and differentiate into myotubes. The biopsy is treated according to an experimental protocol described and validated by us for human skeletal muscle from quadriceps (Kitzmann et al., 2006; El Haddad et al 2017). The biopsy is cut into a fragment of 1 mm³ and placed in a culture dish treated with type 1 collagen. The explants are trapped inside in a thin layer of Matrigel (BD Matrigel Matrix, BD Biosciences) in 35 mm-collagen coated Petri dishes with growth media (DMEM/F12, supplemented with 10 % fetal bovine serum, 0.1% Ultroser G, 1 ng/ml basic FGF, and 10 microg/mL gentamicin). After 6 to 8 days, when cells migrate out of the explants, they are enzymatically harvested using dispase (BD Biosciences) and subcultured in growth medium. Harvested cells are purified by immunomagnetic cell sorting using magnetic activated cell sorter (MACS) microbeads (MiltenyiBiotec) coupled to an antibody against CD56. Usually in protocols of muscle culture cells, muscle differentiation is induced by growing confluent myoblasts in differentiation medium depleted of growth factors. At confluence, myoblasts start to differentiate and differentiation can be evidenced 1) morphologically (after 2 to 4 days), as the fusion of myoblasts generates long giant multinucleated cells (named myotubes) and 2) biochemically, as myotubes express proteins required for muscle contraction. Differentiation is assessed by immunofluorescence using the antibodies against Troponin T and Myogenin, two markers of muscle differentiation. The differentiation status is also confirmed by precisely measuring the expression of three differentiation markers (Myogenin, Sarcomeric Actin and Caveolin by RT-qPCR). Expression of RPLP0 is also quantified as internal control.

Briefly, myoblasts are seeded at 10⁵ cells/dish onto 35 mm collagen-coated dishes and cultured in growth medium (DMEM/F12, supplemented with 10% fetal bovine serum, 0.1% Ultroser G, 1 ng/ml basic FGF, and 10 microg/ml gentamicin). Myogenic differentiation of confluent cells was induced after 3 days by changing to DMEM containing 5% FBS (differentiation medium). Cells were kept in differentiation medium for 3 to 4 days.

Then the cells are fixed and the expression of troponin T (a protein of the cytoskeleton which is exclusively expressed by myotubes) is analysed by immuno-fluorescence. Then the area of myotubes is measured using the Image J software. If the area increases the compound has a hypertrophic activity and if the area decreases the compound has an atrophic activity.

### Hypertrophic activity of the rosemary extract

The hypertrophic activity of hydroalcoholic extract of rosemary leaves was tested in the cell model disclosed above.

The skeletal muscle satellite cells came from young people (aged under 30).

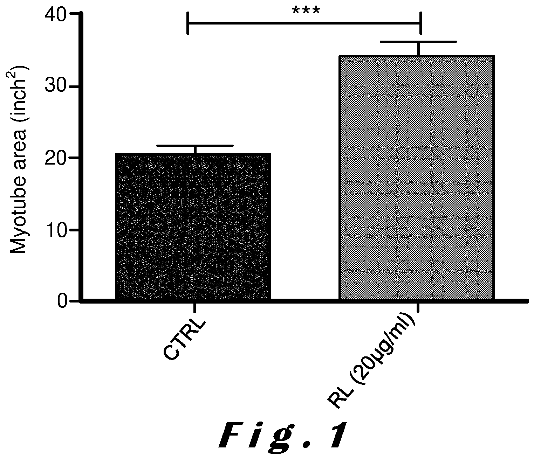

As shown in figure 1, a hydroalcoholic extract of rosemary leaves exerts a hypertrophic activity on myotubes when it is at a concentration of 20 µg/ml in the culture medium.

### Isolation of the hypertrophic compound from the extract of rosemary leaves (RL)

To isolate the compound(s) responsible for the hypertrophic activity of the extract of rosemary leaves, a bioassay-guided fractionation approach was used.

Wild rosemary was harvested in north of Montpellier (France). The dried leaves were crushed and an extraction was carried out directly. 150 g of crushed rosemary leaves were put in obscurity at room temperature in a mix comprising 900 g of ethanol absolute and 450 g of distilled water. The mix was manually agitated every 24 hours. After 7 days maceration, the extract was filtrated. Evaporation was made on dry reduced pression. 69 g of hydroalcoholic extract of rosemary leaves called hereinafter RL was obtained.

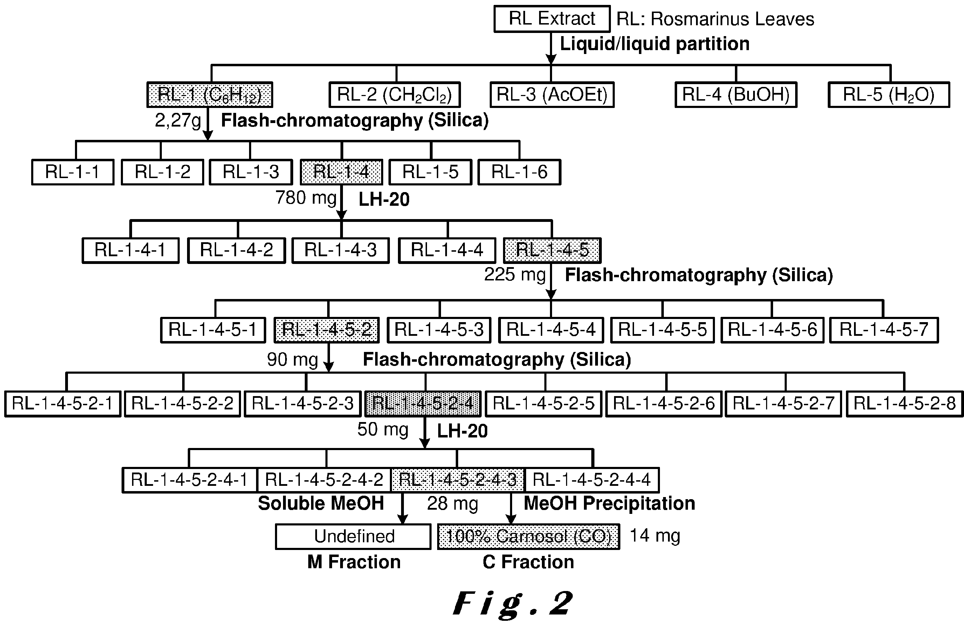

After several purification steps as illustrated in figure 2, an active compound was isolated in the fraction C.

The fraction M also shown a hypertrophic activity but weak compared to fraction C.

A NMR analysis shown that fraction C consists of pure carnosol.

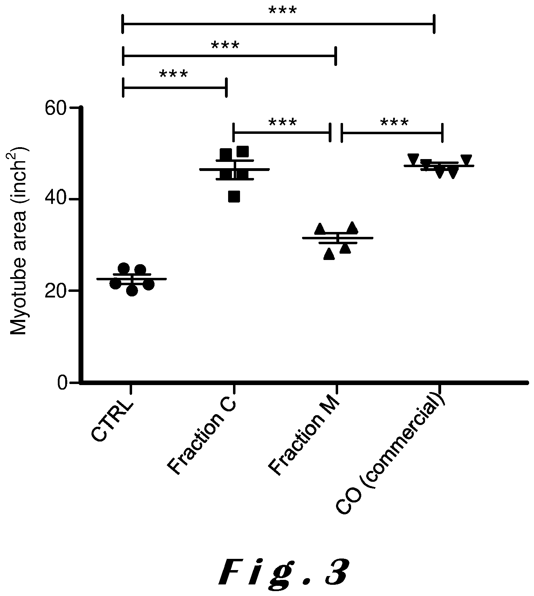

Thus, the hypertrophic compound from the extract of rosemary leaves is the carnosol. In order to confirm this result, the hypertrophic activity of commercially available carnosol was assayed (figure 3). The commercially available carnosol shows the same hypertrophic activity as the fraction C purified from rosemary leaves.

### Carnosol shows a hypertrophic activity from 0.5 µg/ml (1-2 uM)

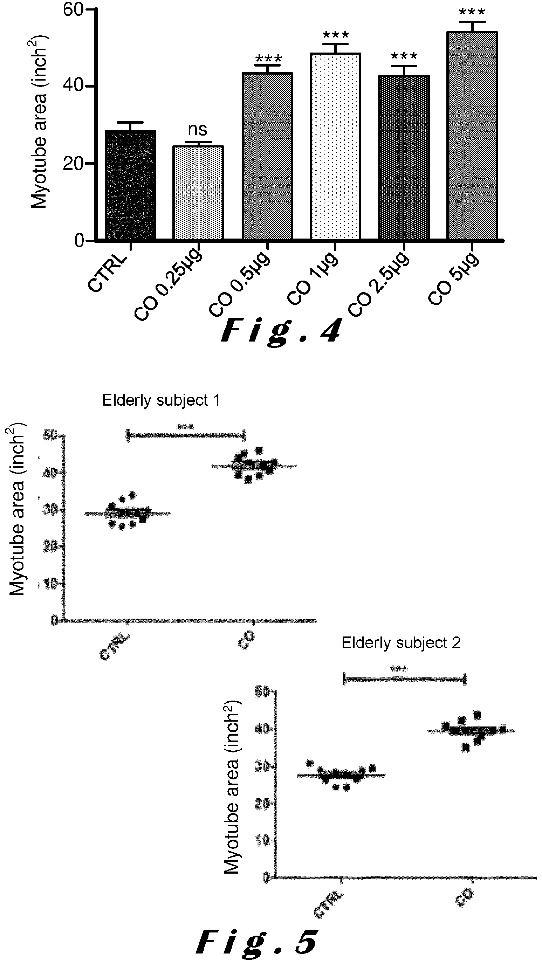

The hypertrophic activity of carnosol was assayed at various concentrations in the culture medium (0.25 µg/ml, 0.5 µg/ml, 1 µg/ml, 2.5 µg/ml and 5 µg/ml). The results are shown in figure 4. The carnosol exhibits a hypertrophic activity from 0.5 µg/ml (i. e. from 1 to 2 µM) in the cell culture model.

### Hypertrophic activity on myotube cells from elderly subject

The culture cell model disclosed above was used with skeletal muscle satellite cells recovered from elderly subjects (subject 1 and subject 2) in order to assay the hypertrophic activity of carnosol on myotube cells from elderly subject.

As shown in figure 5, the carnosol has also a hypertrophic activity on myotube cells of elderly subjects (carnosol: 2µg/ml).

### Hypertrophic activity of carnosol in vivo

To investigate whether carnosol can alter skeletal muscle physiology, adult mice (13 weeks old, C57 BL6 J) were treated for 12 days with a daily oral dose of 80 mg/Kg. Sections were made on gastrocnemius muscle with cryostats followed by staining with an anti-dystrophin antibody, revealed by immunofluorescence. The determination of the fibre areas (CSA) was made using the Myovision software.

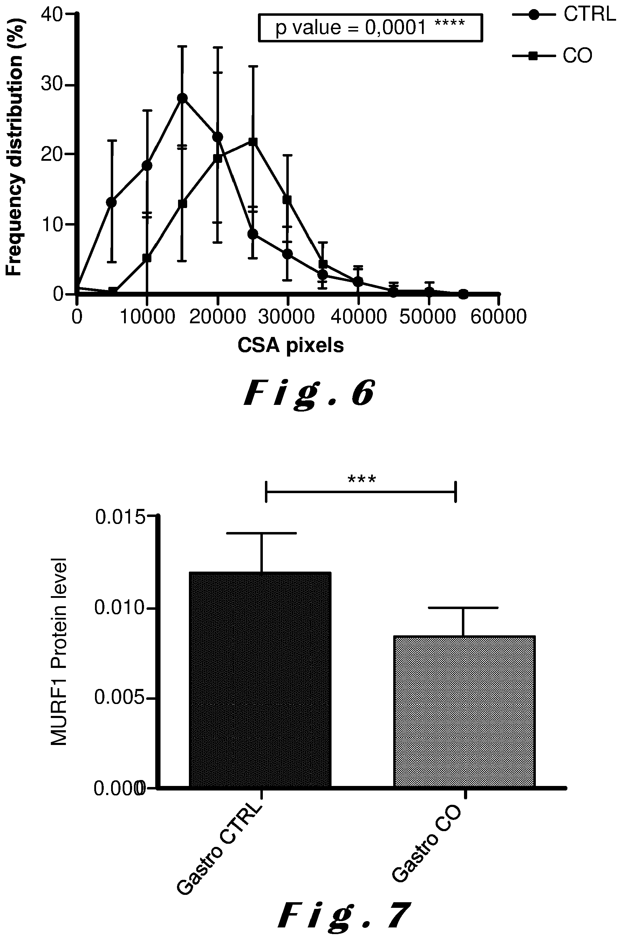

As shown in figure 6, treatment with carnosol significantly increases the diameter of the fibers on the gastrocnemius

Then, a western blot analysis on muscle extract was performed to determine the impact of a carnosol treatment on MURF1 protein. It was found that carnosol down-regulates MURF1 protein level in gastrocnemius from control mice, in good correlation with its impact on gastrocnemius fiber area (figure 7).

### Comparison of the hypertrophic activity of carnosol with other compounds.

The hypertrophic activity of carnosol was compared to other antioxidant compounds.

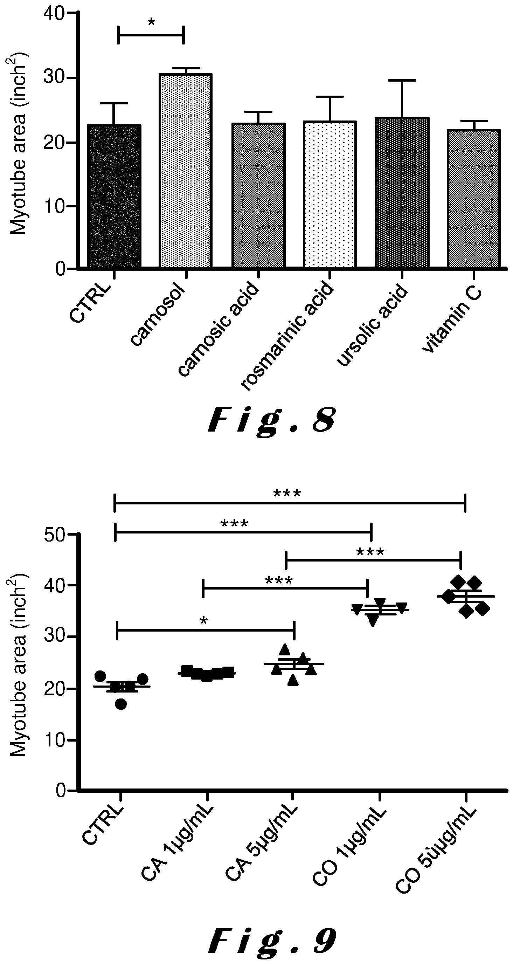

As shown in figure 8, only carnosol has a significant hypertrophic activity. The hypertrophic activity of carnosol goes beyond its antioxidant activity (carnosol: 2µg/ml; carnosic acid: 2 µg/ml; rosmarinic acid: 2 µg/ml; ursolic acid: 2 µg/ml; vitamin C: 50 µg/ml).

Then, the hypertrophic activity of carnosol and carnosic acid was compared. Whereas carnosic acid has a structure very close from the one of carnosol, the hypertrophic activity of carnosol is significantly higher than the one of carnosic acid. This suggests that the hypertrophic activity of carnosol is specific (figure 9).

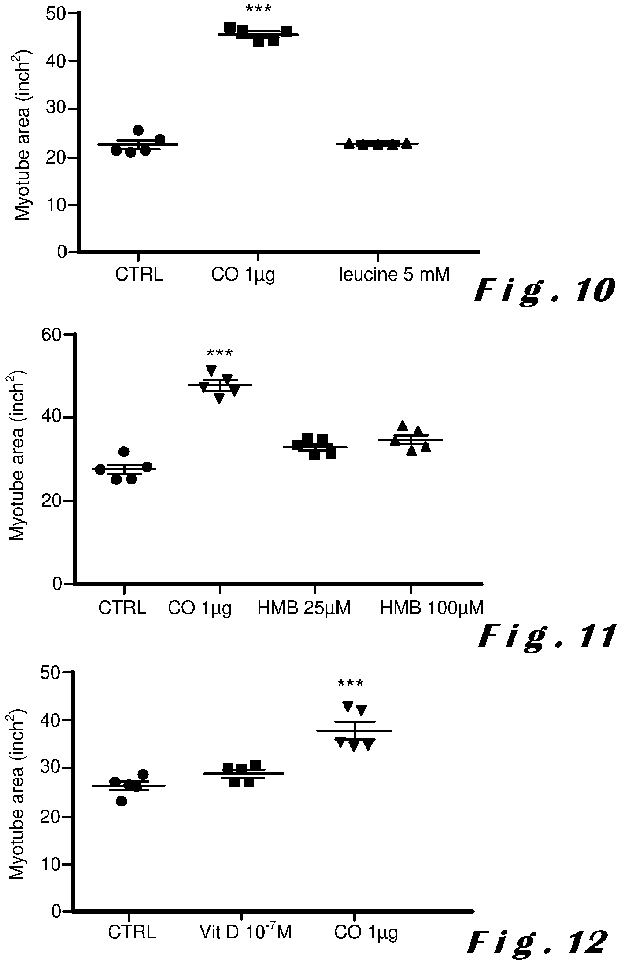

The hypertrophic activity of carnosol was compared to leucine and its metabolite β-hydroxy-β-methylbutyrate known for their anabolic properties. Human myotube were treated for 48 hours with carnosol, leucine and β-hydroxy-β-methylbutyrate. As shown in figures 10 and 11, only carnosol treatment induced myotube hypertrophy.

Low vitamin-D levels are associated with decreased muscle strength and poor physical function in elderly individuals. In addition, it has been proposed that vitamin-D plays an important role for obtaining optimal skeletal muscle function. Thus, the hypertrophic activity of carnosol has been compared to the one of vitamin D. Human myotubes were treated for 48 hours with carnosol or vitamin D in the culture medium at indicated concentrations. Only carnosol induced myotube hypertrophy (figure 12).

### Pathwav(s) involved in the hypertrophic activity of carnosol

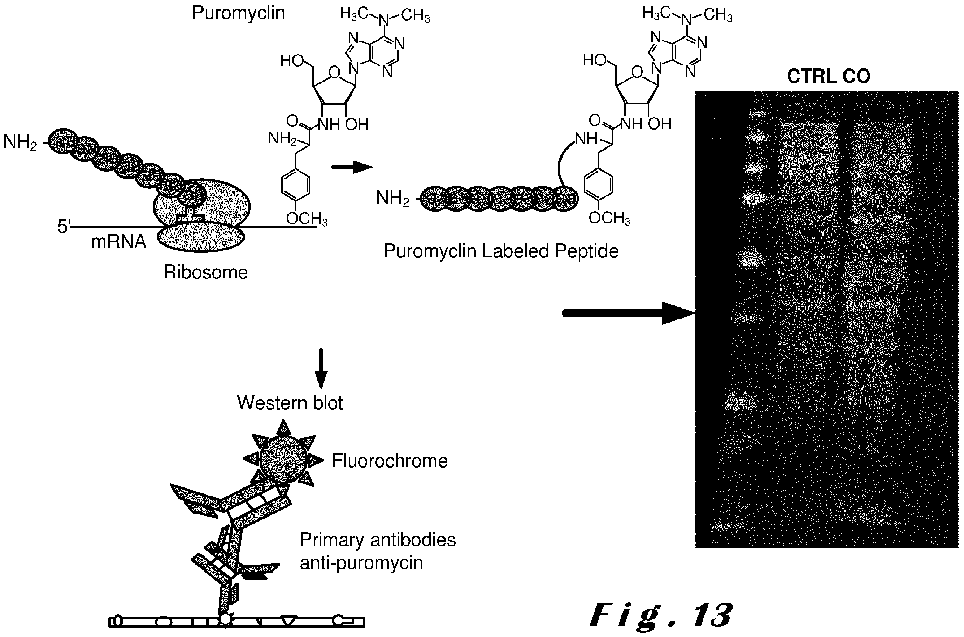

The muscular hypertrophic activity of carnosol may result from a global increase of the synthesis of proteins. To study this hypothesis, the culture cell model disclosed above was stimulated with carnosol. Then, 30 minutes before recovering the protein from the cells, the cells were treated with puromycin. The puromycin is incorporated in the neo-synthetized proteins. Then, a western blot was carried out with anti-puromycin antibodies in order to show the translation rate of the mRNA in the living cells (Schmidt et al., SUnSET, a nonradioactive method to monitor protein synthesis. Nat Methods. 2009;6:275-277) (see figure 13).

Based on these results, the inventors studied if the carnosol is able to regulate various signaling pathways controlling the balance between the protein synthesis and the protein degradation. Two main pathways control the regeneration of contractile proteins:
- PI3K/Akt/mTOR which is the major pathway of muscle hypertrophy and
- pathway of the transcription factors of FOXO family which controls the expression of the genes involved in the proteasome degradation systems and the autophagy (respectively atrogenes and genes of the autophagy).

At the molecular level, it has been established that the activation of the PI3K/Akt/mTOR pathway stimulates the protein synthesis through their anabolizing targets (mTOR, protein S6 kinase 1 (S6K) and the eIF4E-binding proteins (4E-BP) and blocks the ubiquitine E3 of the family of atrogenes MuRF1 and MAFbx mediated proteolysis pathway (FOXO) (Egerman and Glass, Signaling pathways controlling skeletal muscle mass. Crit Rev Biochem Mol Biol. 2014 Jan-Feb;49(1):59-68). According to the available data, the expression of the MuRF1 and MAFbx mRNA is higher in conditions inducing skeletal muscle atrophy (inactivity, denervation, malnutrition, glucocorticoids treatment, oxidative stress, and inflammation). The experiments on mice wherein the expression of MuRF1 or MAFbx was inactivated suggest that MuRF1 would be a better candidate than MAFbx to develop targeted drugs in order to inhibit its expression and thus treat muscular atrophy. Indeed, deleting MuRF1 prevents muscular atrophy in more physiological or physiopathological conditions than the MAFbx deletion. Moreover, the muscular mass which is preserved in response to the MuRF1 deletion seems to be more functional with the strength proportional to the quantity of muscle (Bodine and Baehr, Skeletal muscle atrophy and the E3 ubiquitin ligases MuRF1 and MAFbx/atrogin-1; Am J Physiol Endocrinol Metab. 2014 Sep 15;307(6):E469-84).

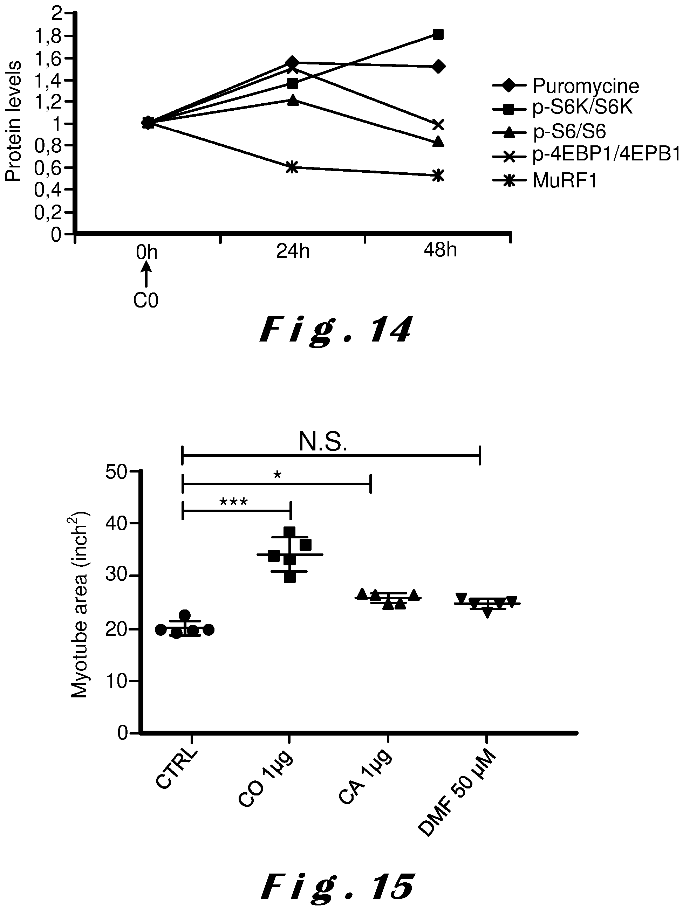

The inventors evaluated with western blot the effects of carnosol on the various signaling pathways involved in the control of skeletal muscle hypertrophy or atrophy and found that carnosol stimulates the mTOR pathway (synthesis of proteins) by increasing the phosphorylation rate of P70 S6 kinase, PS6 and 4EBP1 (see figure 14). Moreover, carnosol inhibits strongly the MuRF1 expression (degradation of proteins) (see figure 14).

The inventors have also studied the NRF2 signaling pathway.

The regulation of Nrf2 signaling is believed to preserve redox homeostasis and protect the structure and function of skeletal muscle. Nrf2 is a transcription factor, inactivated by Keap-1 in the cytoplasm. Upon activation, Keap1 is degraded, and NRF2 mediates intracellular antioxidant response by binding to the antioxidant response element (ARE) in the promoter of its target genes and induces the expression of a set of antioxidant enzymes, called 'phase 2 enzymes,' including heme oxygenase-1 (HO-1). Recently it has been shown that Nrf2 deficiency caused accelerated aging and muscle loss during aging.

Since carnosic acid and carnosol are antioxidants molecules and can activate the NRF2 pathway in several type of cells, it was studied whether carnosic acid and carnosol are capable of inducing NRF2 pathway in skeletal muscle. The study has shown that carnosol (6µM) and carnosic acid (6µM) both induce NRF2 accumulation but only carnosol fully activates NRF2 pathway by inducing HO-1.

In order to know whether NRF2 pathway activation is sufficient to activate hypertrophy in skeletal muscle cells. An assay with DMF (dimethyl Fumarate) has been carried out. Given DMF is a NRF2 activator, if the NRF2 pathway activation is sufficient to activate hypertrophy in skeletal muscle cells then DMF should induce myotube hypertrophy.

The assay has shown that DMF induces the NRF2 pathway (NRF2 and HO-1) in skeletal muscle cells but DMF is a poor inductor of NRF2, efficient at doses superior to 20 µM whereas carnosol induces the NRF2 pathway from 3 µM.

Moreover, DMF treatment does not induce repression of MURF1 protein and myotube hypertrophy in contrast to carnosol treatment (figure 15).

## Claims

1. Non therapeutic use of carnosol for increasing muscle protein synthesis and/or for reducing muscle protein degradation in a subject.

2. Non therapeutic use of carnosol for preventing and/or treating loss of muscle mass and/or for increasing muscle mass in a subject.

3. Non therapeutic use of carnosol according to claim 1 or 2 wherein the subject is selected from the group consisting of a subject having a sedentary lifestyle, a subject at bed rest or having been at bedrest, an immobilized subject, an undernourished subject, a malnourished subject and an astronaut.

4. Non therapeutic use of carnosol or according to claim 1 or 2 wherein the subject is a sportsperson.

5. Carnosol for use in the prevention and/or the treatment of muscle atrophy, in particular sarcopenia, and/or muscular dystrophy in a subject.

6. Carnosol for use according to claim 5 wherein the subject is an elderly subject.

7. Carnosol for use according to claim 5 wherein the muscle atrophy is a muscle atrophy associated with a disease selected from the group consisting of cancer, AIDS, congestive heart failure, COPD (chronic obstructive pulmonary disease), renal failure, trauma, sepsis, severe burns, mental disease such as anorexia, neuronal disease, cachexia, obesity and drug-related iatrogenia.

8. A non therapeutic method for increasing muscle protein synthesis and/or for reducing muscle protein degradation in a subject in need thereof comprising the step of administering carnosol to said subject.

9. A non therapeutic method for increasing muscle mass of a livestock animal comprising the step of administering carnosol to said animal.

10. Non therapeutic use of a composition comprising carnosol for preventing and/or treating loss of muscle mass and/or for increasing muscle mass in a subject wherein the composition comprises at least 0.1% w/w of carnosol.

11. The non therapeutic use according to claim 10, wherein the composition comprises at least 2.5% w/w of carnosol.

12. A composition comprising carnosol for use in the prevention and/or the treatment of muscle atrophy and/or muscular dystrophy in a subject wherein the composition comprises at least 0.1% w/w of carnosol, preferably the muscle atrophy being sarcopenia.

13. The composition comprising carnosol for use according to claim 12, wherein the composition comprises at least 2.5% w/w of carnosol.

14. A non therapeutic method for increasing muscle mass of a livestock animal comprising the step of administering to said animal a composition comprising carnosol wherein the composition comprises at least 0.1% w/w of carnosol.

15. The non therapeutic method for increasing muscle mass of a livestock animal according to claim 14, wherein the composition comprises at least 2.5% w/w of carnosol.

## Patentansprüche

1. Nicht-therapeutische Verwendung von Carnosol zur Steigerung der Muskelproteinsynthese und/oder zur Verringerung des Muskelproteinabbaus bei einem Individuum.

2. Nicht-therapeutische Verwendung von Carnosol zur Vorbeugung und/oder Behandlung des Verlustes von Muskelmasse und/oder zur Steigerung der Muskelmasse bei einem Individuum.

3. Nicht-therapeutische Verwendung von Carnosol nach Anspruch 1 oder 2, wobei das Individuum ausgewählt ist aus der Gruppe bestehend aus einem Individuum mit Bewegungsmangel, einem Individuum, das unter Bettruhe steht oder unter Bettruhe stand, einem gelähmten Individuum, einem unterernährten Individuum, einem mangelernährten Individuum und einem Astronauten.

4. Nicht-therapeutische Verwendung von Carnosol nach Anspruch 1 oder 2, wobei es sich bei dem Individuum um einen Sportler handelt.

5. Carnosol zur Verwendung in der Vorbeugung und/oder der Behandlung von Muskelatrophie, insbesondere Sarkopenie, und/oder Muskeldystrophie bei einem Individuum.

6. Carnosol zur Verwendung nach Anspruch 5, wobei es sich bei dem Individuum um ein älteres Individuum handelt.

7. Carnosol zur Verwendung nach Anspruch 5, wobei es sich bei der Muskelatrophie um eine Muskelatrophie
handelt, die mit einer Erkrankung assoziiert ist, ausgewählt aus der Gruppe bestehend aus Krebs, AIDS, kongestiver Herzinsuffizienz, COPD (Chronic Obstructive Pulmonary Disease, chronisch obstruktive Lungenerkrankung), Niereninsuffizienz, Trauma, Sepsis, schweren Verbrennungen, psychischer Erkrankung wie etwa Anorexie, neuronaler Erkrankung, Kachexie, Fettleibigkeit und arzneimittelbedingter latrogenie.

8. Nicht-therapeutisches Verfahren zur Steigerung der Muskelproteinsynthese und/oder zur Verringerung des Muskelproteinabbaus bei einem Individuum, das dessen bedarf, welches den Schritt des Verabreichens von Carnosol an das Individuum umfasst.

9. Nicht-therapeutisches Verfahren zur Steigerung der Muskelmasse bei einem Nutztier, das den Schritt des Verabreichens von Carnosol an das Tier umfasst.

10. Nicht-therapeutische Verwendung einer Carnosol umfassenden Zusammensetzung zur Vorbeugung und/oder Behandlung eines Verlustes von Muskelmasse und/oder zur Steigerung der Muskelmasse bei einem Individuum, wobei die Zusammensetzung mindestens 0,1 % w/w Carnosol umfasst.

11. Nicht-therapeutische Verwendung nach Anspruch 10, wobei die Zusammensetzung mindestens 2,5 % w/w Carnosol umfasst.

12. Carnosol umfassende Zusammensetzung zur Verwendung in der Vorbeugung und/oder der Behandlung von Muskelatrophie und/oder Muskeldystrophie bei einem Individuum, wobei die Zusammensetzung mindestens 0,1 % w/w Carnosol umfasst, wobei es sich bei der Muskelatrophie vorzugsweise um Sarkopenie handelt.

13. Carnosol umfassende Zusammensetzung zur Verwendung nach Anspruch 12, wobei die Zusammensetzung mindestens 2,5 % w/w Carnosol umfasst.

14. Nicht-therapeutisches Verfahren zur Steigerung der Muskelmasse bei einem Nutztier, das den Schritt des Verabreichens einer Carnosol umfassenden Zusammensetzung an das Tier umfasst, wobei die Zusammensetzung mindestens 0,1% w/w Carnosol umfasst.

15. Nicht-therapeutisches Verfahren zur Steigerung der Muskelmasse bei einem Nutztier nach Anspruch 14, wobei die Zusammensetzung mindestens 2,5 % w/w Carnosol umfasst.

## Revendications

1. Utilisation non thérapeutique de carnosol pour augmenter la synthèse des protéines musculaires et/ou pour réduire la dégradation des protéines musculaires chez un sujet.

2. Utilisation non thérapeutique de carnosol pour prévenir et/ou traiter la perte de masse musculaire et/ou pour augmenter la masse musculaire chez un sujet.

3. Utilisation non thérapeutique de carnosol selon la revendication 1 ou 2, dans laquelle le sujet est choisi dans le groupe constitué par un sujet ayant un mode de vie sédentaire, un sujet alité ou ayant été alité, un sujet immobilisé, un sujet sous-alimenté, un sujet mal nourri et un astronaute.

4. Utilisation non thérapeutique de carnosol selon la revendication 1 ou 2 dans laquelle le sujet est un sportif.

5. Carnosol pour une utilisation dans la prévention et/ou le traitement de l'atrophie musculaire, en particulier la sarcopénie, et/ou la dystrophie musculaire chez un sujet.

6. Carnosol pour une utilisation selon la revendication 5, dans lequel le sujet est un sujet âgé.

7. Carnosol pour une utilisation selon la revendication 5, dans lequel l'atrophie musculaire est une atrophie musculaire associée à une maladie choisie dans le groupe constitué par le cancer, le SIDA, l'insuffisance cardiaque congestive, la BPCO (bronchopneumopathie chronique obstructive), l'insuffisance rénale, un traumatisme, une septicémie, des brûlures graves, une maladie mentale telle que l'anorexie, une maladie neuronale, la cachexie, l'obésité et l'iatrogénie médicamenteuse.

8. Procédé non thérapeutique pour augmenter la synthèse des protéines musculaires et/ou pour réduire la dégradation des protéines musculaires chez un sujet qui en a besoin, comprenant l'étape consistant à administrer du carnosol audit sujet.

9. Procédé non thérapeutique pour augmenter la masse musculaire d'un animal d'élevage comprenant l'étape d'administration de carnosol audit animal.

10. Utilisation non thérapeutique d'une composition comprenant du carnosol pour prévenir et/ou traiter la perte de masse musculaire et/ou pour augmenter la masse musculaire chez un sujet, dans laquelle la composition comprend au moins 0,1% p/p de carnosol.

11. Utilisation non thérapeutique selon la revendication 10, dans laquelle la composition comprend au moins 2,5% p/p de carnosol.

12. Composition comprenant du carnosol pour une utilisation dans la prévention et/ou le traitement de l'atrophie musculaire et/ou de la dystrophie musculaire chez un sujet, dans laquelle la composition comprend au moins 0,1% p/p de carnosol, de préférence l'atrophie musculaire étant la sarcopénie.

13. Composition comprenant du carnosol pour une utilisation selon la revendication 12, dans laquelle la composition comprend au moins 2,5% p/p de carnosol.

14. Procédé non thérapeutique pour augmenter la masse musculaire d'un animal d'élevage comprenant l'étape consistant à administrer audit animal une composition comprenant du carnosol, dans lequel la composition comprend au moins 0,1% p/p de carnosol.

15. Procédé non thérapeutique pour augmenter la masse musculaire d'un animal d'élevage selon la revendication 14, dans lequel la composition comprend au moins 2,5% p/p de carnosol.
